# DEMANDE DE BREVET EUROPEEN

(11) **EP 3 257 585 A1**
(43) Date de publication de la demande: **20.12.2017**
(21) Numéro de dépôt: 16192227.3
(22) Date de dépôt: 04.10.2016
(51) Int. Cl.: B01L 3/02

(54) **EMBOUT ET DISPOSITIF POUR LE PRÉLÈVEMENT DE COLONIES DE MICROORGANISMES ET PROCÉDÉ DE PRÉLÈVEMENT LES METTANT EN OEUVRE**

(30) Priorité: 16.06.2016 EP 16174789
(71) Demandeur: bioMérieux, 69280 Marcy-L'Etoile (FR)
(72) Inventeur: BEAULIEU, Corinne, 69480 Lachassange (FR); CHARRIER, Jean-Philippe, 69160 Tassin la Demi-Lune (FR); FOUCAULT, Frédéric, 69280 Marcy l'Etoile (FR); PARIS, Cécile, 69690 Bessenay (FR); WANDELS, Philippe, 69003 Lyon (FR); COLIN, Bruno, 69280 Marcy l'Etoile (FR); MAKROUF, Bouchra, 69009 Lyon (FR)

(57) **Abrégé**

La présente invention concerne un embout apte à être emmanché sur le corps d'un dispositif de prélèvement, manuel ou automatisé, de matière biologique d'origine microbienne, comprenant :
a) une extrémité distale comprenant un moyen de prélèvement de matière biologique d'origine microbienne,
b) une extrémité libre proximale destinée à entrer en contact avec le corps dudit dispositif de prélèvement et permettre la fixation dudit embout sur ledit corps,
ledit embout étant caractérisé en ce que toute ou partie de l'extrémité libre distale est constitué d'un matériau fibreux présentant une porosité au moins égale à 30 %.

## Description

Le domaine technique de la présente invention est celui des dispositifs destinés à venir prélever des colonies de microorganismes sur milieu de culture gélosé aux fins d'analyse. Plus particulièrement, la présente invention concerne un embout de prélèvement présentant dans sa partie distale une extrémité de prélèvement en matériaux polymères. L'invention concerne également un dispositif de prélèvement comportant un tel embout et un procédé de prélèvement les mettant en oeuvre.

Actuellement, le prélèvement d'une colonie de microorganismes (bactéries, moisissures, levures ou similaires) cultivés sur milieu de culture gélosé en boîte de Pétri, ou sur tout autre support, est réalisé à l'aide d'outils stérilisables ou d'outils à usage unique tels qu'oeses (ou anses), bâtonnets, tubes ou encore cônes.

Cependant, ces outils ne permettent pas de prélever de façon sûre et efficace tous les types de microorganismes car ces derniers peuvent revêtir des formes, tailles, consistances, structures ou aspects très divers.

D'autre part, ces consommables ne permettent pas facilement un dépôt optimal de la matière biologique prélevée sur des supports d'analyse tels que des plaques destinées à une analyse en spectrométrie de masse de type MALDI-TOF. Par ailleurs, il est également très important de pouvoir prélever une colonie bactérienne ou une fraction de cette colonie, sans prélever le milieu de culture situé sous la colonie. Ceci peut en effet fausser les résultats d'analyses ultérieures.

La qualité des résultats d'analyse peut dépendre également de la concentration du dépôt en matière biologique, formé à partir de l'échantillon prélevé, ainsi que de son homogénéité sur le support sur lequel il est déposé. C'est particulièrement le cas pour les analyses de microorganismes en MALDI-TOF, dans lesquelles l'échantillon doit former une couche fine et uniforme pour permettre une analyse optimale.

De manière traditionnelle, il est fait usage d'oeses jetables ou stérilisables à la flamme, pour prélever une colonie de microorganismes sur boite de Pétri et déposer le matériel biologique sur la plaque de MALDI-TOF. Cette opération n'est pas facile et demande une certaine dextérité. La préhension d'un tel outil entre le pouce et l'index peut occasionner des troubles musculo-squelettiques. L'oese est généralement tenue loin de son extrémité pour que la main de l'opérateur ne contamine pas l'échantillon, mais cette position de la main rend plus délicate la précision du mouvement au bout de l'oese, notamment lorsqu'il est nécessaire de réaliser un dépôt fin et homogène sur une petite surface de l'ordre de quelques mm². Enfin l'oese, ayant été dessinée pour prélever de façon calibrée une quantité donnée de microorganismes (généralement entre 1µL et 10µL), comporte à son extrémité une boucle de métal ou de plastique qui a un diamètre généralement supérieur à 1mm. Cette pointe peut être plus grosse que la surface sur laquelle doit être réalisée le dépôt. De plus, la nature rigide de l'oese (métallique ou plastique) n'est pas particulièrement adaptée à l'étalement sur une surface dure d'une colonie microbienne en une fine couche uniforme.

Il est également possible d'utiliser d'autres consommables tels que des écouvillons, des bâtonnets en bois, des embouts de micropipette.

Ainsi, le document US 9,181,522 décrit une méthode et un appareil pour le transfert aseptique de matière biologique. L'appareil se compose d'une chambre double-parois pour loger des embouts de taille unique présentant une bille en tête, destinée au transfert du matériel biologique d'un endroit à un autre, de façon aseptique. Un tel dispositif présente comme premier inconvénient d'être de conception relativement complexe, avec un système de stérilisation des embouts par UV intégré, son architecture double-parois et son système de chargement interne et d'éjection des embouts. Un telle complexité se répercute indubitablement sur le prix de revient et donc de vente. Par ailleurs, le matériau utilisé pour réaliser la bille présente en tête d'embout jetable est en matériau dur, du type métal ou polypropylène, qui n'est pas propice au processus de prélèvement mais surtout de dépôt de matériel biologique, telle qu'une colonie bactérienne, notamment sur une plaque d'analyse en spectrométrie de masse, du type MALDI-TOF.

Le document FR 2 668 495 décrit un cône de prélèvement stérile à usage bactériologique. Ledit cône présente à son extrémité distale une protubérance pleine et légèrement tronconique, désaxée par rapport à l'axe longitudinal du cône. Cette protubérance permet le prélèvement de matériel biologique. Elle peut présenter à cette fin, selon un mode particulier de réalisation, une anse optionnelle. Même avec une architecture particulière, le cône décrit dans ce document n'en demeure pas moins constitué par un matériau classiquement utilisé pour ce genre de produit. A savoir un matériau plastique dur et lisse qui n'est pas adapté au prélèvement et au dépôt de matière biologique. Par ailleurs, sa forme particulière ne rend pas son utilisation aisée pour le dépôt de matière biologique, telle qu'un colonie bactérienne sur une surface très réduite, telle qu'une plaque d'analyse de spectrométrie de masse MALDI-TOF.

La demanderesse a précédemment résolue tout ou partie des inconvénients mentionnés ci-dessus en proposant un procédé de prélèvement de tout ou partie d'un échantillon de matière biologique cultivé au contact d'un milieu de culture gélosé, qui utilise une sonde pourvue d'une extrémité de terminaison. Ledit procédé de prélèvement est essentiellement basé sur le refroidissement de l'extrémité de terminaison de la sonde, permettant le collage de tout ou partie de l'échantillon de matière biologique à prélever par contact de l'extrémité de terminaison sur l'échantillon de matière biologique ou par l'application d'une pression exercée par l'extrémité de terminaison sur l'échantillon de matière biologique, puis le relargage de tout ou partie de l'échantillon de matière biologique par réchauffement de l'extrémité de terminaison de la sonde. Ce procédé est décrit dans la demande de brevet WO 2012/004545.

Ce procédé présente comme principal inconvénient de nécessiter un appareillage de refroidissement de la sonde relativement encombrant, qui consomme de l'énergie et représente un coût financier important.

Il ressort de l'analyse de l'état de la technique, qu'il n'existe pas à ce jour de système de prélèvement de matériel biologique, facile à utiliser, simple de conception et mettant en oeuvre un embout de prélèvement jetable ayant des propriétés physiques adaptées pour optimiser non seulement le prélèvement mais également le dépôt de matière biologique telle qu'une colonie bactérienne.

Les objectifs de la présente invention sont donc de répondre à ces manques en proposant un embout simple de conception, facile à produire, permettant lorsqu'il est placé sur un dispositif de prélèvement de matière biologique de prélever et déposer avec précision cette matière biologique, notamment sur une plaque d'analyse de spectrométrie de masse, de type MALDI-TOF.

Ces objectifs parmi d'autres sont atteints par la présente invention qui concerne en premier lieu, un embout apte à être emmanché sur le corps d'un dispositif de prélèvement, manuel ou automatisé, de matière biologique d'origine microbienne, comprenant :
a) une extrémité distale comprenant un moyen de prélèvement de matière biologique d'origine microbienne,
b) une extrémité libre proximale destinée à entrer en contact avec le corps dudit dispositif de prélèvement et permettre la fixation dudit embout sur ledit corps,
ledit embout étant caractérisé en ce que toute ou partie de l'extrémité libre distale est constitué d'un matériau fibreux présentant une porosité au moins égale à 30 %.

Selon un mode avantageux, l'embout selon l'invention est constitué entièrement d'un matériau fibreux présentant une porosité au moins égale à 30 %.

De façon préférentielle, le matériau fibreux présente une porosité supérieure à 50%, préférentiellement supérieure à 70%.

Avantageusement, le matériau fibreux est pris dans le groupe comprenant : polyéthylène, polyesters, polytéréphtalate d'éthylène (PET), copolymère PET/polyéthylène, copolymère PET/PET, polyamide, coton.

L'embout de prélèvement présente une forme globale sensiblement conique ou tronconique. Le moyen de prélèvement est quant à lui avantageusement de forme globale cylindrique, tronconique ou sphérique.

Un autre objet de la présente invention concerne un dispositif de prélèvement de matière biologique d'origine microbienne comprenant :
un embout selon l'invention
un corps comportant au moins :
   une partie proximale servant au moins partiellement de zone de préhension dudit dispositif et
   une partie distale présentant une extrémité libre au bout de laquelle est fixé ledit embout.

Ce dispositif comporte en outre un système d'éjection de l'embout. Avantageusement, le système d'éjection comporte une tige positionnée à l'intérieur dudit corps et mobile en translation, de manière à venir faire pression sur l'embout et ainsi l'éjecter.

Selon un mode de réalisation particulier, ladite tige est mobile en translation au moyen d'un bouton poussoir.

Un autre objet de l'invention concerne un procédé de prélèvement de matière biologique comprenant les étapes suivantes :
a) Positionner un embout selon l'invention sur la partie distale du dispositif de prélèvement,
b) Positionner le dispositif de prélèvement à proximité d'une colonie de matière biologique d'origine microbienne présente sur un milieu de culture afin de mettre en contact le moyen de prélèvement dudit embout avec la matière biologique,
c) Prélever tout ou partie de la matière biologique à l'aide du dispositif de prélèvement, de sorte que la matière biologique prélevée soit solidaire du moyen de prélèvement.

Un autre objet de la présente invention concerne un procédé de préparation d'une plaque d'analyse pour une analyse microbiologique en spectrométrie de masse de type MALDI-TOF à partir d'une échantillon de matière biologique comprenant les étapes suivantes :
a) Positionner un embout sur la partie distale du dispositif de prélèvement, selon l'invention
b) Positionner le dispositif de prélèvement à proximité d'une colonie de matière biologique d'origine microbienne présente sur un milieu de culture afin de mettre en contact le moyen de prélèvement de l'embout avec la matière biologique,
c) Prélever tout ou partie de la matière biologique à l'aide du dispositif de prélèvement, de sorte que la matière biologique prélevée soit solidaire du moyen de prélèvement,
d) Réaliser un dépôt uniforme de matière biologique prélevée sur au moins une zone d'analyse d'une plaque d'analyse de spectrométrie de masse de type MALDI-TOF, par mise en contact du moyen de prélèvement avec la surface de ladite au moins une zone d'analyse.

Selon un mode de réalisation particulier, ce procédé comporte en outre une étape supplémentaire e) d'éjection de l'embout de prélèvement.

Les buts et avantages de la présente invention seront mieux compris à la lumière de la description détaillée et nullement limitative de l'invention, qui suit, faite en référence au dessin dans lequel :
La figure 1A représente une vue en perspective d'un embout de prélèvement selon la présente invention, selon un premier mode de réalisation.
La figure 1B représente une vue de côté de l'embout représenté sur la figure 1A.
La figure 1C représente une vue en perspective d'un embout de prélèvement selon un second mode de réalisation
La figure 2A représente une vue de côté d'un dispositif de prélèvement de matière biologique, selon un premier mode de réalisation.
La figure 2B représente une vue éclatée de côté du dispositif de prélèvement de matière biologique, représenté sur la figure 2A.
La figure 2C représente une vue en perspective du corps du dispositif de prélèvement de matière biologique tel que représenté sur le figure 2A.
La figure 2D représente une vue de côté d'un dispositif de prélèvement de matière biologique tel que représenté sur le figure 2A, dans la configuration d'éjection de l'embout de prélèvement.
La figure 3A représente une vue de côté d'un dispositif de prélèvement de matière biologique, selon un deuxième mode de réalisation.
La figure 3B représente une vue éclatée de côté du dispositif de prélèvement de matière biologique, représenté sur la figure 3A.
La figure 3C représente une vue en perspective, agrandie et éclatée du système de fixation de l'anneau-poussoir sur la tige du dispositif de prélèvement représenté sur la figure 3A.
La figure 4A représente une vue de côté d'un dispositif de prélèvement de matière biologique, selon un troisième mode de réalisation.
La figure 4B représente une vue éclatée de côté du dispositif de prélèvement de matière biologique, représenté sur la figure 4A.
La figure 4C représente une vue agrandie du mécanismes d'éjection de l'embout du dispositif de prélèvement représenté sur la figure 4A.
La figure 5A représente une vue en perspective d'un dispositif de prélèvement de matière biologique, selon un quatrième mode de réalisation.
La figure 5B représente une vue éclatée en perspective du dispositif de prélèvement de matière biologique, représenté sur la figure 5A.
La figure 5C représente une vue en perspective du dispositif de prélèvement de matière biologique, représenté sur la figure 5A, en configuration d'éjection de l'embout de prélèvement.

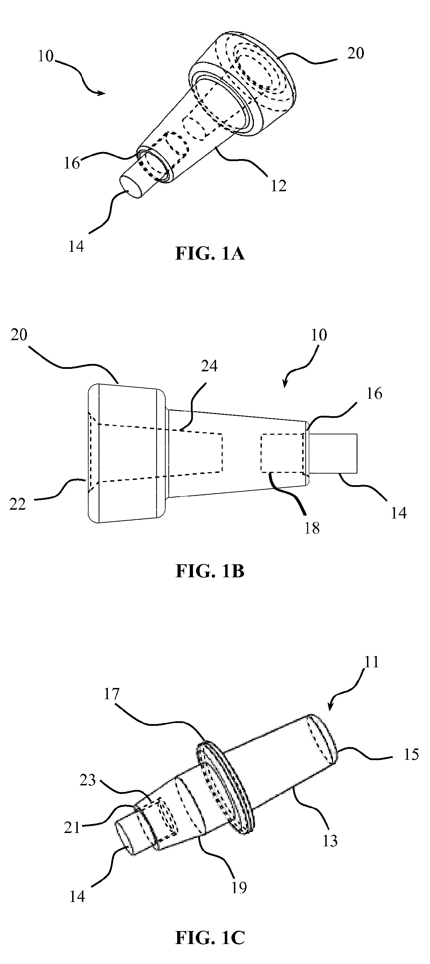

Sur la figure 1A, l'embout 10 selon un premier mode de réalisation est représenté selon une vue en perspective. Il est représenté en vue de côté, sur la figure 1B. Selon ce mode de réalisation, l'embout 10 est de forme générale tronconique. Il est néanmoins tout à fait envisageable que l'embout 10 selon l'invention soit de forme différente. Il est constitué de trois parties distinctes. En premier lieu, une partie distale 12 de forme sensiblement tronconique. Cette partie distale 12 constitue la partie de réception d'un moyen de prélèvement 14. A cette fin, la partie distale 12 présente une extrémité libre 16, au niveau de laquelle est ménagée une cavité borgne 18, dans laquelle est positionné le moyen de prélèvement 14. Dans ce but, la forme de la cavité 18 et celle du moyen de prélèvement 14 doivent être complémentaires. Idéalement, les dimensions du moyen de prélèvement 14 doivent être légèrement inférieures à celle de la cavité 18 pour permettre son insertion. Toutefois, il est préférable qu'elles soient assez proches pour éviter que le moyen de prélèvement ne se désolidarise de la cavité. L'embout 10 comporte par ailleurs une partie proximale 20, également de forme tronconique mais moins longue et plus large que la partie distale 12. L'extrémité 22 de la partie proximale 20 est libre et présente une cavité borgne 24 à l'intérieur de laquelle vient se loger l'extrémité distale d'un dispositif de prélèvement.

L'embout 10 peut être moulé dans les matériaux habituellement utilisés pour mouler les embouts de pipettes. Le matériau peut être par exemple un polymère de type polyoléfine. Ce type de matériau est généralement bon marché, stérilisable et adapté à une utilisation pour la réalisation de produit à usage unique.

Concernant le moyen de prélèvement 14, le matériau constituant ce dernier a toute son importance. En effet, le moyen de prélèvement a comme double contrainte technique de devoir assurer un prélèvement de matière biologique, telle qu'une colonie bactérienne mais également un relargage de ladite matière biologique lors de son dépôt sur un dispositif d'analyse tel qu'une plaque d'analyse de spectrométrie de masse. Les inventeurs ont ainsi découvert que la porosité du matériau utilisé pour fabriquer le moyen de prélèvement 14 était une caractéristique essentielle pour permettre de trouver le bon compromis entre les performances de prélèvement et les performances de relargage de matière biologique.

Par ailleurs, au-delà des caractéristiques intrinsèques du moyen de prélèvement, il est important d'avoir à l'esprit que lorsque la matière biologique à prélever est une colonie bactérienne, la façon dont cette dernière se comporte lors de son prélèvement ou lors de son dépôt peut varier d'une espèce bactérienne à l'autre. Il est en effet bien connu que les colonies bactériennes sont plus ou moins consistantes, plus ou moins visqueuses, plus ou moins fileuses selon l'espèce bactérienne considérée. Il est donc indispensable de disposer d'un moyen de prélèvement qui soit apte à manipuler n'importe quel type de colonie bactérienne, quel que soit ses propriétés.

Ainsi les inventeurs ont identifié qu'une porosité au moins égale à 30% était nécessaire pour obtenir les propriétés recherchées. Idéalement une porosité au moins égale à 70% permet d'obtenir les meilleurs résultats.

Par porosité du matériau, on entend l'ensemble des vides (pores) d'un matériau solide pouvant être remplis par des fluides (liquide ou gaz). Nous entendrons également par porosité la grandeur physique définie comme le rapport entre le volume des vides et le volume total du milieu poreux étudié.

La mesure de porosité du matériau est réalisée de la façon suivante :
Un échantillon du matériau fibreux sec est prélevé.

L'échantillon est pesé une première fois.

L'échantillon est immergé dans l'eau, le temps nécessaire à son imprégnation totale.

L'échantillon imprégné est un nouvelle fois pesé.

La masse de l'eau emprisonnée dans le matériau est déduite par calcul de la différence entre la masse du matériau imprégné et celle du matériau sec.

La masse volumique de l'eau étant proche de 1, il en est déduit le volume d'eau emprisonnée et ainsi le volume des vides dans le matériau.

Le volume de l'échantillon imprégné est alors mesuré.

En divisant la valeur de vides dans l'échantillon de matériau, obtenue ci-dessus par le volume mesuré de l'échantillon imprégné, il est ainsi possible d'obtenir la valeur de la porosité du matériau.

Les matériaux particulièrement adaptés pour réaliser le moyen de prélèvement peuvent être des matériaux fibreux. Parmi les matériaux, on peut citer les matériaux synthétiques tel que les polyéthylènes, les polyesters, les polyamides. Parmi les polyesters, on peut citer le polytéréphtalate d'éthylène (PET). Il peut s'agir également de copolymères, tels que des polymères de polyéthylène/polyester, tel qu'un copolymère de polyéthylène/ PET ou encore un copolymère PET/PET. Lorsqu'il s'agit d'un matériau fibreux, les fibres peuvent être constituées d'un matériau mono-composant ou bi-composant. Une fibre bi-composant peut être par exemple constituée d'un coeur en PET et d'une gaine en polyéthylène.

Des matériaux fibreux naturels peuvent également être utilisés. C'est le cas notamment des fibres de coton.

Un second mode de réalisation de l'embout est représenté sur la figure 1C. Cet embout 11 est constitué de trois parties : une partie proximale 13 sensiblement cylindrique ou éventuellement tronconique. Contrairement au premier mode de réalisation, l'extrémité libre 15 de la partie proximale 13 ne présente pas de cavité borgne et est bouchée. En effet, selon ce mode de réalisation, l'extrémité distale du dispositif de prélèvement ne vient pas s'insérer dans l'embout 11. C'est au contraire l'embout 11 qui vient s'insérer dans l'extrémité distale du dispositif de prélèvement, et plus précisément son extrémité proximale 15. Bien entendu, pour ce faire, le dispositif de prélèvement doit présenter une extrémité distale libre susceptible de recevoir l'embout 11. Il doit ainsi présenter une cavité de réception de l'embout 11 dont les dimensions doivent être légèrement supérieures aux dimensions de la partie proximale 13. L'embout 11 présente également une collerette 17 qui permet l'éjection dudit embout après utilisation. Elle peut également servir de butée lorsque l'embout 11 est inséré dans le dispositif de prélèvement. Enfin l'embout 11 présente une partie distale 19 destinée à recevoir un moyen de prélèvement 14, comme dans le cas de l'embout 10, selon le premier mode de réalisation. A cette fin, la partie distale 19 présente une extrémité libre 21, au niveau de laquelle est ménagée une cavité borgne 23, dans laquelle est positionné le moyen de prélèvement 14.

Selon une variante de réalisation de l'embout de prélèvement 11, l'extrémité proximale 13 peut présenter un orifice permettant de générer un trou traversant entre les extrémités distale et proximale. Il est alors possible d'utiliser un moyen de prélèvement plus long, positionnée dans le trou traversant et solidaire de l'embout au niveau de l'orifice de l'extrémité proximale, voire au niveau des deux extrémités. Une telle variante est plus simple de conception et donc moins couteuse à produire.

Le moyen de prélèvement 14, tel qu'il est représenté sur les figures 1A à 1C est de forme cylindrique. Il est néanmoins possible que le moyen de prélèvement 14 présente une forme différente. Ainsi, l'extrémité libre de ce dernier peut être par exemple conique pour améliorer encore la précision de prélèvement et de dépôt du matériel biologique. L'extrémité libre de l'embout 14 peut également présenter toute autre forme adaptée au prélèvement de matière biologique.

Selon une variante de l'invention, il pourrait être envisagé d'avoir un embout de prélèvement qui soit constitué intégralement du matériau poreux. Selon cette variante de réalisation, l'embout de prélèvement constituerait également le moyen de prélèvement.

Selon une autre variante de l'invention, le moyen de prélèvement, l'embout de prélèvement et le dispositif de prélèvement pourraient ne faire qu'un. En effet, il peut être envisagé un dispositif de prélèvement à usage unique, auquel est solidarisé un embout de prélèvement poreux. Alternativement, le dispositif de prélèvement peut être entièrement fabriqué en matériau poreux. Pour ce faire, les dimensions du moyen de prélèvement doivent permettre une préhension et une manipulation aisées.

L'embout de prélèvement conjugué au moyen de prélèvement ou le moyen de prélèvement seul, s'il ne fait qu'un avec l'embout de prélèvement, peuvent être adaptés pour être utilisés avec un système automatisé de prélèvement de matière biologique. Un tel système pourrait par exemple réaliser de manière automatique le prélèvement de colonies bactériennes sur des boites de Pétri et la préparation de plaques d'analyse en spectrométrie de masse.

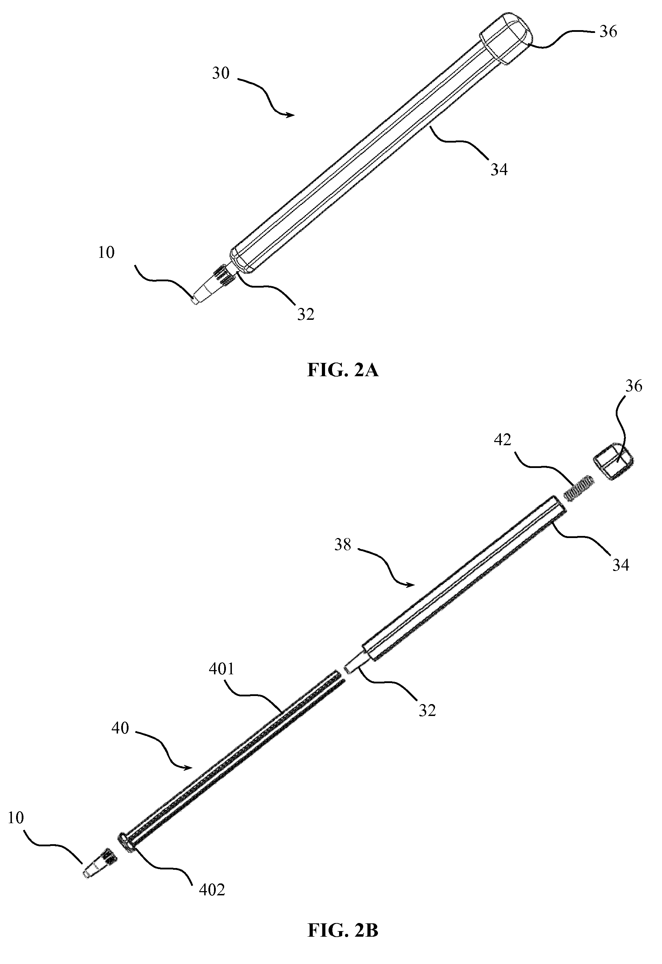

Sur la figure 2A, est représenté un dispositif de prélèvement 30 selon un premier mode de réalisation. Ce dispositif de prélèvement 30 comporte une partie distale 32, une partie intermédiaire 34 et une partie proximale 36. La partie distale 32, sensiblement tronconique, a pour fonction de porter l'embout de prélèvement 10 selon l'invention. La partie intermédiaire 34, également cylindrique, a pour fonction de permettre la préhension du dispositif de prélèvement 30 par son utilisateur. Enfin, la partie proximale 36 a une forme générale de godet, dont le diamètre intérieure est légèrement supérieur au diamètre extérieur de la partie intermédiaire 34, de tel sorte que la partie proximale peut partiellement recouvrir l'extrémité proximale de la partie intermédiaire.

La figure 2B représente le dispositif de prélèvement 30 en vue éclatée de telle sorte que chaque élément constitutif de ce dispositif est représenté séparément. On constate ainsi que les parties distale 32 et intermédiaire 34 sont en fait une seule et même pièce 38 constituant le corps du dispositif de prélèvement.

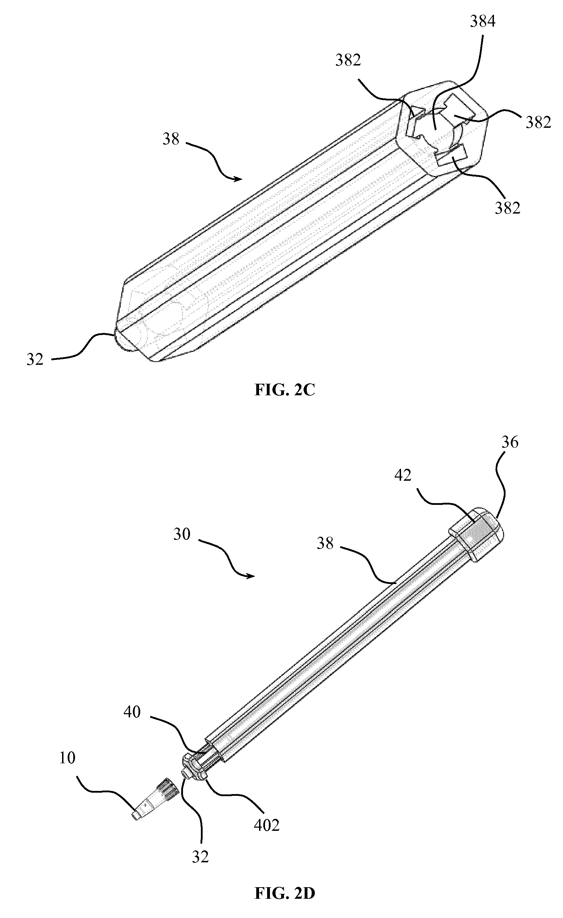

Tel que représenté sur le figure 2C, ce corps 38 sensiblement cylindrique présente trois évidements 382 longitudinaux traversants et périphériques, ainsi qu'un évidement central 384. Les évidements 382 sont ménagés afin de recevoir chacun une lame 401 de la tige 40, tel que représenté sur la figure 2B. Quant à l'évidement 384, il est ménagé pour recevoir un ressort hélicoïdal 42, comme représenté sur la figure 2B. La tige 40 est donc constituée principalement de trois lames 401 dont la base est solidarisée à un anneau-butoir 402. La tige 40 vient se positionner à l'intérieur du corps du dispositif 38 en introduisant les extrémités libres des lames 401 dans les évidements 382, jusqu'à ce que l'anneau-butoir 402 vienne en appuie contre l'extrémité distale de la partie intermédiaire 34, alors que la partie distale 32 vient traverser la lumière dudit anneau-butoir 402. A l'extrémité opposée du corps 38, l'extrémité inférieure du ressort hélicoïdal 42 vient se loger dans l'évidement 384, alors que son extrémité supérieure est placée en contact de la face intérieure de la partie proximale 36 du dispositif de prélèvement, qui vient coiffer le corps 38. Cette partie proximale joue le rôle de poussoir, lors de l'éjection de l'embout de prélèvement 10, une fois ce dernier utilisé.

Les lames 401 présentent une longueur supérieure à la partie intermédiaire 34 de telle sorte que lorsque la tige 40 est positionnée à l'intérieur du corps 38, l'extrémité libre supérieure des lames 401 émerge des évidements 382. Ces extrémités libres sont solidarisés avec le poussoir 36, une fois le ressort en place. Cette solidarisation peut être mécanique. Ainsi, il est envisageable de disposer d'ergots en saillie radiale à proximité de l'extrémité libre des lames 401 qui viennent se loger dans des évidements ménagés dans la paroi interne du poussoir 36. Alternativement, il est possible de solidariser l'extrémité libre des lames 401 avec la face interne du poussoir 36 par collage chimique.

Comme représenté sur la figure 2D, lorsqu'une pression dans l'axe longitudinal du dispositif de prélèvement est exercée sur le poussoir 36, ce dernier vient coulisser sur le corps 38. Cette action de coulissement se répercute sur la tige 40 qui est solidaire du poussoir 36 et coulisse à l'intérieur du corps 38. Ce coulissement se manifeste à l'extrémité distale du dispositif de prélèvement par une translation de l'anneau-poussoir 402. Comme ce dernier est en appui contre l'extrémité proximale de l'embout 10, il entraîne en translation ledit embout de telle sorte que ce dernier est désolidarisé de l'extrémité distale 32 du dispositif de prélèvement 30. La pression exercée par le poussoir 36 sur le ressort hélicoïdal 42 entraîne la compression de ce dernier. Cette compression se répercute sur la tige 40, qui coulisse tel qu'expliqué supra. Lorsque la pression sur la partie proximale 36 est relâchée, le ressort 42 tend à reprendre une position de repos dans laquelle il ne fait plus pression sur la tige 40. Cette dernière reprend alors sa position initiale par translation inverse, libérant la partie distale 32 qui est alors disponible pour recevoir un autre embout de prélèvement.

Les différentes étapes du procédé de mise en oeuvre du dispositif de prélèvement 30 avec un embout de prélèvement 10 peuvent donc être résumées ainsi :
Un embout 10 est positionné sur l'extrémité libre 32 du dispositif de prélèvement. Cette étape est généralement réalisée en venant positionné le dispositif en position vertical en aplomb d'un embout stérile, généralement positionnée sur un portoir adapté. L'extrémité de la partie distale 32 du dispositif de prélèvement est alors emmanchée dans la cavité 24 de la partie proximale 20 de l'embout de 10, jusqu'à ce qu'elle soit en butée au fond de la cavité 24.

Pour réaliser un prélèvement de matière biologique telle qu'une colonie de microorganismes sur un milieu de culture gélosé, le dispositif de prélèvement est positionné de telle manière que le moyen de prélèvement de l'embout vienne en contact de la colonie de microorganismes. Tout ou partie de la colonie est alors prélevée.

Pour réaliser le dépôt de tout ou partie de la matière biologique prélevée, sur une surface telle que la surface d'une plaque d'analyse de spectrométrie de masse MALDI-TOF, le dispositif de prélèvement est approché de ladite surface, de telle sorte que l'extrémité du moyen de prélèvement portant la matière biologique vienne en contact de la surface. De légers mouvements circulaires sont alors réalisés avec le dispositif de prélèvement pour déposer une couche de matière biologique sur ladite surface.

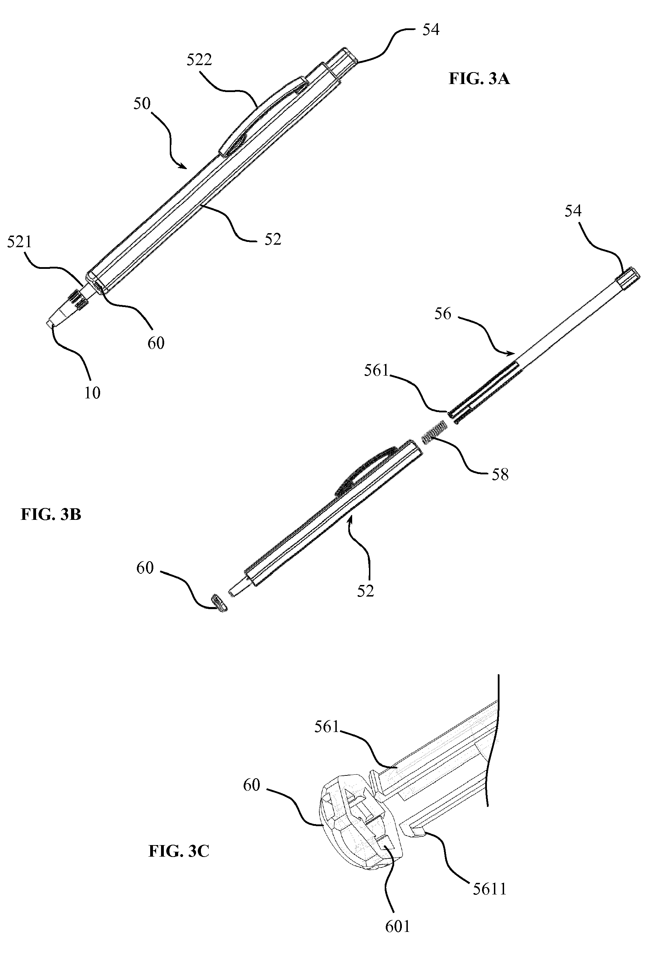

Une fois le dépôt réalisé, l'embout de prélèvement est éjecté en actionnant le moyen d'éjection et jeté dans une poubelle. Dans le cas du dispositif de prélèvement tel que représenté sur les figures 3 à 5, l'éjection est réalisée en exerçant une pression sur la partie proximale 36.

Un second mode de réalisation du dispositif de prélèvement est représenté sur les figures 3A à 3C. Ce dispositif de prélèvement 50 est constitué d'un corps 52 sensiblement cylindrique et creux. Ce corps 52 présente en sa partie distale une extrémité libre 521 de forme sensiblement tronconique, destinée à porter un embout de prélèvement 10. Cette extrémité libre 521 est solidarisée au corps 52 par trois ailettes (non représentées) définissant trois espaces interstitiels. Le corps 52 présente également un système d'accrochage ou agrafe 522 permettant d'accrocher le dispositif de prélèvement à la poche d'un vêtement par exemple, comme on peut le faire avec un stylo. Enfin, sur la figure 3A, on observe également un bouton poussoir 54 qui sort par l'extrémité proximale du corps 52. Comme on peut le voir sur la figure 3B, le bouton poussoir 54 est solidaire d'une tige 56, prenant place à l'intérieur du corps 52. Cette tige 56 de forme sensiblement cylindrique se termine à son extrémité distale par trois pattes de fixation 561, représentées en gros plan sur le vue agrandie 3C. L'espace central entre lesdites pattes de fixation 561 est destiné à recevoir un ressort hélicoïdal 58, tel que montré sur la figure 3B.

La tige 56 est insérée dans le corps 52 par l'extrémité proximale de ce dernier, le ressort hélicoïdal 58 étant préalablement inséré. Ledit ressort hélicoïdal 58 vient se positionner en appui contre les ailettes et la partie supérieure de l'extrémité distale 521. La tige 56, une fois insérée, vient se positionner à cheval sur le ressort de telle sorte que les pattes de fixation 561 viennent traverser les espaces interstitiels définis entre les ailettes et ressortir à la base du corps 52 autour de l'extrémité libre 521. Une bague 60 est insérée autour de l'extrémité libre 521. Cette bague 60 dispose des trois évidements 601 destinés à recevoir l'extrémité des pattes de fixation 561. Les pattes de fixation disposent à leur extrémité d'ergots radiaux 5611, qui une fois les pattes positionnées à l'intérieur de la bague 60, vont venir se verrouiller dans les évidements 601 et ainsi solidariser la bague 60 et la tige 56. A titre d'alternative et dans un souci de simplifier le mécanisme, il est tout à fait possible de remplacer ce système de solidarisation mécanique par un système comportant des pattes sans ergots qui seraient solidarisées à la bague par collage. Comme représenté sur le figure 3A, la bague 60 vient en appui contre le corps 52 au niveau de la partie supérieure de l'extrémité libre 521, du fait de l'action du ressort hélicoïdal 58 qui vient exercer sur la tige 56, une force de répulsion vers la partie supérieure du corps 52.

Comme dans le cas du premier mode de réalisation du dispositif de prélèvement 30, lorsqu'une pression dans l'axe longitudinal du dispositif de prélèvement est exercée sur le bouton poussoir 54, cette pression se répercute sur la tige 56, solidaire du bouton poussoir 54. Cette dernière vient alors coulisser à l'intérieur du corps 52, comprimant alors le ressort hélicoïdal 58. Ce coulissement se manifeste à l'extrémité distale du dispositif de prélèvement par une translation de la bague 60, de sorte que cette dernière vient appuyer sur l'extrémité proximale de l'embout 10 et entraîner ce dernier en translation jusqu'à ce qu'il soit désolidarisé de l'extrémité distale 521 du dispositif de prélèvement 50. Lorsque la pression exercée par la tige 56 sur le ressort hélicoïdal 58 est relâchée, du fait du relâchement de la pression sur le bouton poussoir 54, ce dernier reprend sa position de repos. La tige 56 est entraînée en translation inverse par pression du ressort, jusqu'à ce que la bague 60 revienne en appui contre le corps 52, rendant la partie distale 521 à nouveau accessible pour recevoir un autre embout de prélèvement 10.

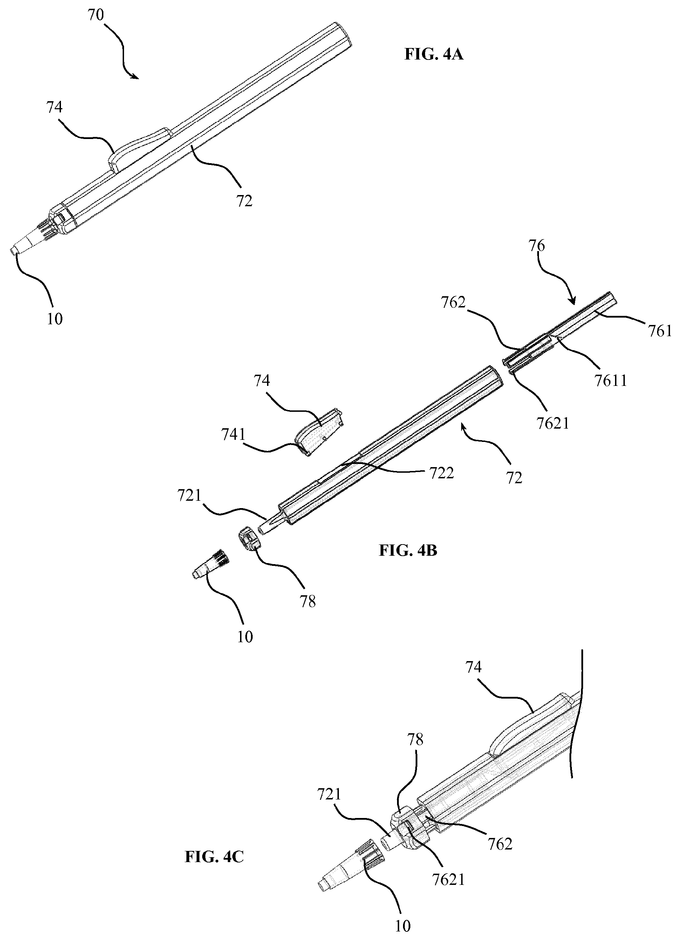

Un troisième mode de réalisation d'un dispositif de prélèvement est représenté sur les figure 4A à 4C. Ce dispositif de prélèvement 70 est constitué d'un corps 72 sensiblement cylindrique et creux. Ce corps 72 présente en sa partie distale une extrémité libre 721 de forme sensiblement tronconique, destinée à porter un embout de prélèvement 10. Cette extrémité libre 721 est solidarisée au corps 72 par des trois ailettes (non représentées) définissant trois espaces interstitiels. On observe également un bouton poussoir 74 positionné latéralement sur le corps 72, qui vient s'insérer dans un logement 722 ménagé dans la paroi latérale du corps 72, comme on peut le voir sur la figure 4B. Il comporte également une tige 76 qui est insérée dans le corps 72 par son extrémité supérieure. Cette tige 76 est constituée d'une partie proximale monobloc 761 de section transversale sensiblement rectangulaire et d'une partie distale constituée de trois pattes de fixation 762. A l'interface avec la partie distale, la partie proximale 761 présente sur ces deux faces opposées un épaulement incliné 7611, destiné à venir coopérer avec le bouton poussoir 74. La tige 76 est insérée dans le corps 72 du dispositif de prélèvement 70, par l'extrémité proximale de ce dernier. Une fois la tige 76 insérée, les pattes de fixation 762 viennent traverser les espaces interstitiels définis entre les ailettes de maintien de l'extrémité libre 721 et ressortir à la base du corps 72 autour de ladite extrémité libre 721. Une bague 78 est insérée autour de l'extrémité libre 721. Cette bague 78 est similaire à la bague 60 décrite supra et dispose des trois évidements destinés à recevoir l'extrémité des pattes de fixation 762, également pourvus d'ergots radiaux 7621 et destinés à coopérer avec les évidements de la bagues 78 pour solidariser la tige 76 à la bague 78, tel que montrés sur la figure 4C.

Le bouton poussoir 74 a une section transversale en forme de « U » inversé, destinée à lui permettre de se positionner à cheval autour de la partie proximale 761 de la tige 76, particulièrement au niveau des épaulements inclinés 7611. Le bouton poussoir 74 présente sur chacune de ces deux faces latérales trois ergots 741 destinés à l'empêcher de ressortir, une fois positionné dans le logement 722. Lorsque le tige 76 et le bouton poussoir 74 sont positionnés dans le corps 72 du dispositif de prélèvement 70, le bouton poussoir 74 vient en appui au niveau de sa partie distale contre les épaulements inclinés 7611, empêchant la tige 76 de ressortir. L'interaction entre le bouton poussoir et les épaulements inclinés 7611 permet le déplacement de la tige 76 entre une position de repos et une position d'éjection de l'embout de prélèvement 10. En effet, lorsqu'un embout 10 est positionné sur l'extrémité distale 721 du dispositif de prélèvement 70, ce dernier vient en appui contre la bague 78. Cette dernière est alors positionnée en position haute, contre le corps 72, tel que montré sur le figure 4A. La tige est alors en position de repos, dans laquelle le bouton poussoir 74 se retrouve en position haute.

Lorsque l'utilisateur du dispositif de prélèvement 70 souhaite éjecter l'embout de prélèvement 10, il exerce une pression sur le bouton poussoir 74. Cette pression entraine l'enfoncement du bouton poussoir dans le logement 722, qui glisse alors sur les épaulements inclinés 7611. Le bouton poussoir ne pouvant se mouvoir latéralement, son glissement sur les épaulements inclinés 7611 entraîne le déplacement de la tige 76 en translation horizontale vers l'extrémité distale du corps 72. La tige passe alors de sa position de repos vers sa position d'éjection de l'embout, dans laquelle la bague d'éjection 78 se retrouve en position basse après avoir exercé une pression sur l'embout et entrainer l'éjection de ce dernier, comme représenté sur la figure 4C.

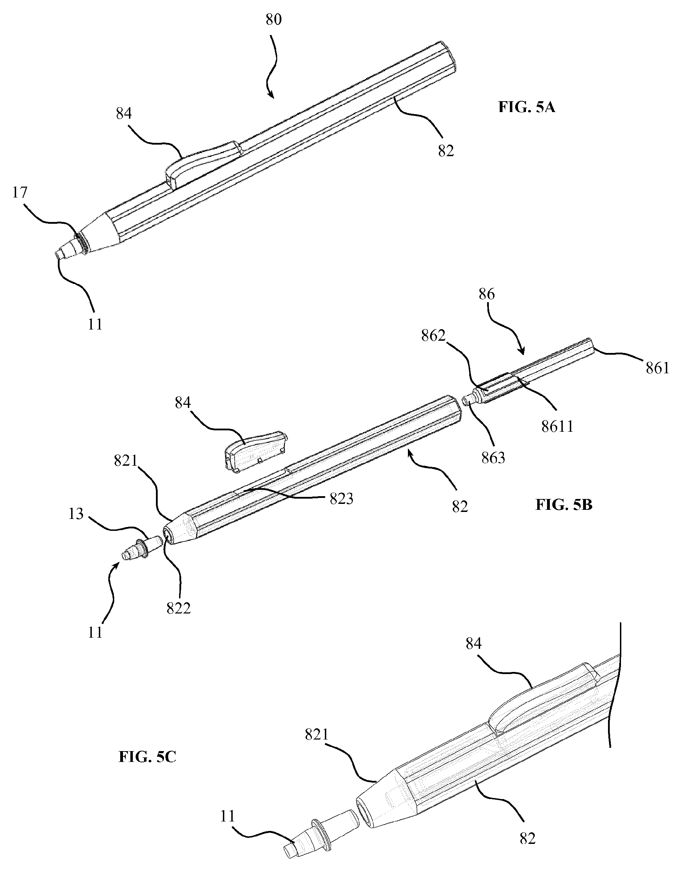

Un quatrième mode de réalisation est représenté sur les figures 5A et 5C. Ce mode de réalisation est très similaire au troisième mode de réalisation pour ce qui concerne le fonctionnement du dispositif de prélèvement. En effet, le dispositif de prélèvement 80 représenté sur les figures 5A à 5C est constitué d'un corps 82 sensiblement cylindrique et creux. Ce corps 82 est monobloc et présente en sa partie distale une extrémité libre 821 de forme sensiblement tronconique. Cette extrémité 821 est percée d'un orifice circulaire 822. Cet orifice est destiné à recevoir la partie proximale 13 d'un embout de prélèvement 11, tel que décrit en lien avec la figure 1C. Dans cette configuration, l'embout de prélèvement 11 est enfoncé dans le corps du dispositif de prélèvement 80 jusqu'à ce que la collerette 17 de l'embout 11 vienne en appui contre l'extrémité 821 du corps 82. Le corps 82 comporte également un bouton poussoir 84 positionné latéralement sur le corps 82, qui vient s'insérer dans un logement 823 ménagé dans la paroi latérale du corps 82. Il comporte enfin une tige 86 qui est insérée dans le corps 82 par son extrémité supérieure. Cette tige 86 est constituée d'une partie proximale monobloc 861 de section transversale sensiblement rectangulaire, d'une partie distale monobloc 862 de section transversale sensiblement ronde se terminant par un téton 863. A l'interface entre la partie proximale 861 et la partie distale 862 sont ménagées de épaulements inclinées 8611 de part et d'autre de la partie proximale 861. Ces épaulements 8611 et le bouton poussoir 84 sont amenés à coopérer selon un mécanisme identique à celui qui a été décrit supra en lien avec le dispositif de prélèvement 70 représenté sur les figures 4A à 4C, de sorte que lorsque l'utilisateur appuie sur le bouton poussoir 84, il déclenche le déplacement en translation de la tige 86 dans la lumière du corps 82, vers l'extrémité distale de ce dernier. Le téton 863 vient alors en appui contre l'extrémité libre 15 de la partie proximale 13 de l'embout 11 et exerce une pression sur ce dernier de sorte que l'embout 11 est éjecté du dispositif de prélèvement 80 par translation. Ceci est bien montré sur la figure 5C. Lors du positionnement d'un nouvel embout 11 sur le dispositif de prélèvement 80, la partie proximale 13 qui pénètre dans la lumière du corps 82 par l'orifice 822 vient pousser la tige 86 en sens opposé, entraînant la remontée du bouton poussoir 84 par translation verticale grâce aux épaulements inclinés 8611 qui coopèrent avec le bouton poussoir 84.

D'autres modes de réalisation du dispositif de prélèvement sont envisageables. Ainsi, un autre mode de réalisation possible consiste à positionner à l'extrémité distale du corps mais sur sa partie extérieure, un système mécanique destiné à venir en appui contre l'embout pour permettre son éjection. Il peut s'agir par exemple d'un manchon mobile en translation sur le corps du dispositif de prélèvement qui est en contact avec l'embout de prélèvement et qui permet d'éjecter ce dernier quand il est déplacé par l'utilisateur.

D'autres protocoles de préparation d'échantillons de matière biologique en vue d'une analyse peuvent être envisagés avec le dispositif de prélèvement. Ainsi, une fois le prélèvement de matière biologique effectué, il est possible de préparer une suspension de matière biologique dans une solution ad hoc, telle qu'une solution saline. Pour ce faire, il peut être envisagé de libérer l'embout de prélèvement portant la matière biologique, directement dans un tube contenant une quantité de solution de remise en suspension. Une agitation sous vortex du tube contenant l'embout permettra alors la libération de la matière biologique retenue sur le matériau fibreux.

### EXEMPLES :

### Exemple 1: Utilisation d'un dispositif de prélèvement avec des embouts de prélèvement pour la préparation d'une plaque en vue d'une analyse par MALDI-TOF.

Le dispositif de prélèvement mis en oeuvre dans cet exemple est le dispositif représenté sur les figures 2A à 2D et décrit *supra*.

L'embout de prélèvement est en matériau plastique, tel que ceux utilisés classiquement pour réaliser les embouts de pipette jetable. Il comporte un moyen de prélèvement de forme cylindrique d'une diamètre 2 mm et d'une longueur de 6mm. Il est fibres de copolymère polytéréphtalate d'éthylène (PET)/PET, matériau commercialisé par la société Porex sous la référence PSU-832.

Le corps du dispositif de prélèvement est réalisé en polypropylène injecté. Il présente une longueur de 120 mm et une section de forme hexagonale avec 8,2 mm entre les faces opposées de l'hexagone, ce qui permet une préhension aisée comme avec un crayon, sans risque de troubles musculo-squelettiques.

Le dispositif de prélèvement est utilisé pour prélever des colonies de différentes espèces bactériennes, qui ont poussé sur milieu de culture Columbia + 5% sang de mouton (COS), commercialisé par la demanderesse sous la référence 43041.

Les colonies prélevées sont déposées sur une plaque d'analyse MALDI-TOF jetable à 48 positions, commercialisée par la demanderesse sous la référence 410893.

Parmi les espèces choisies, certaines sont connues pour former des colonies qui sont difficiles à prélever. C'est le cas par exemple de *Bacillus lichenformis, Klebsiella pneumoniae, Proteus mirabilis* ou encore *Nocardia asteroides.*

Le procédé est réalisé selon les étapes suivantes :
1. Un embout de prélèvement est positionné sur un dispositif de prélèvement.
2. Une colonie bactérienne est prélevée sur un milieu de culture de type COS, à l'aide du dispositif de prélèvement en mettant en contact le moyen de prélèvement en matériau fibreux porté sur l'embout de prélèvement, avec la colonie.
3. L'échantillon bactérien prélevé est déposé sur la plaque d'analyse MALDI-TOF, sur l'une des 48 positions prévues à cet effet, en mettant en contact le moyen de prélèvement portant l'échantillon bactérienne avec la plaque MALDI-TOF, en exerçant une pression et en étalant l'échantillon. La pression exercée sur le matériau fibreux du moyen de prélèvement permet de retenir l'excès d'échantillon sur ledit matériau fibreux. Il est possible de réaliser des dépôts successifs sur différentes positions de la plaque d'analyse dans le cas où l'on souhaite répliquer l'analyse. Ces autres dépôts peuvent se faire avec le même embout.
4. Une fois l'ensemble des dépôts réalisé, l'embout de prélèvement est éjecté dans une poubelle en exerçant une pression sur le bouton poussoir, tel que décrit *supra.*
5. 1 µL de matrice (acide alpha cyano 4-hydroxycinnamique prêt à l'emploi, bioMérieux référence 411071) est déposé sur chaque dépôt de matière biologique, à l'aide d'une micropipette.
6. Les étapes 1 à 5 sont renouvelées pour chaque colonie que l'on souhaite analyser.
7. Le dépôt d'une souche *d'E. coli* ATCC 8739, utilisée comme calibrant et contrôle positif, est également réalisé conformément aux étapes 1 à 5.
8. La plaque d'analyse est stockée le temps nécessaire au séchage de la matrice.
9. Une fois la plaque entièrement sèche, celle-ci est introduite dans le spectromètre de masse MALDI-TOF VITEK® MS, commercialisé par la demanderesse, l'acquisition des spectres de masse est réalisée.
10. L'identification des microorganismes est obtenue par analyse des spectres à l'aide du système expert et de la base de données VITEK® MS.

L'analyse des mêmes colonies bactériennes est réalisée en mettant en oeuvre le procédé traditionnel de préparation de plaque d'analyse MALDI-TOF mettant en oeuvre une oese et bien connu de l'homme du métier.

Les résultats obtenus avec les deux procédés de préparation de plaque d'analyse sont regroupés dans le **TABLEAU 1** et comparés.

Le symbole « ✔ » signifie que le spectre est de bonne qualité et a permis l'identification de l'espèce attendue.

Le symbole « X » signifie que le spectre n'est pas de qualité suffisante pour pemettre l'identification de l'espèce.

**TABLEAU 1**

| **Espèce** | **Protocole de référence utilisant une oese** | **Protocole utilisant le dispositif selon l'invention** |
|---|---|---|
| *B. lichenformis (souche API 0608016)* | X | ✔ |
| *E. aerogenes (souche ATCC 13048)* | ✔ | ✔ |
| *E. coli (souche ATCC 25922)* | X | ✔ |
| *E. faecalis (souche API 1006023)* | ✔ | ✔ |
| *K. oxytoca (souche API 1006024)* | X | ✔ |
| *K. pneumoniae (souche ATCC 13883)* | X | ✔ |
| *K. pneumoniae ssp ozaenae (souche API 1006025)* | X | ✔ |
| *N. asteroides (souche API 1201096)* | ✔ | ✔ |
| *N. farcinica (souche API 1201094)* | X | ✔ |
| *P aeruginosa (souche ATCC 27853)* | ✔ | ✔ |
| *P. mirabilis (souche ATCC 12453)* | X | ✔ |
| *S. aureus (souche ATCC 29213)* | ✔ | ✔ |
| *S. epidermidis (souche ATCC 12228)* | ✔ | ✔ |
| Temps nécessaire au prélèvement et au dépôt des échantillons | 16 minutes | 12 minutes |

Il ressort de l'analyse du tableau 1 qu'en utilisant le protocole mettant en oeuvre le dispositif de prélèvement selon l'invention, toutes les espèces analysées sont correctement identifiées en spectrométrie de masse avec le VITEK® MS.

Par comparaison, avec le protocole traditionnel mettant en oeuvre une oese pour prélever les colonies bactériennes, seules 6 espèces sur 13 analysées sont correctement identifiées. Parmi les espèces non identifiées, une majorité sont celles connues pour former des colonies difficiles à manipuler.

De façon particulièrement avantageuse, le procédé de l'invention permet donc de prélever et de déposer des espèces bactériennes formant des colonies avec une consistance atypique. Ainsi, il est possible d'obtenir facilement d'excellent résultats d'identification par spectrométrie de masse MALDI-TOF pour des espèces pulvérulentes et incrustantes comme *B. lichenformis* ou *N. farcinica,* des espèces gluantes comme *K. pneumoniae* et des espèces diffusant sur la gélose comme *P. mirabilis*.

On constate par ailleurs que le temps nécessaire au dépôt de 48 échantillons est plus court avec le dispositif de prélèvement selon l'invention (12 min) qu'avec une oese (16 min).

Ainsi le procédé de la présente invention est plus ergonomique, plus facile à mettre en oeuvre, plus rapide et permet d'obtenir de meilleurs résultats que le procédé de référence de l'art antérieur.

### Exemple 2 : Comparaison de la performance d'identification des différentes espèces bactériennes par analyse en spectrométrie de masse, avec différents matériaux fibreux

Dans cet exemple, plusieurs matériaux fibreux présentant des porosités différentes ont été testés, notamment dans leur capacité à permettre un prélèvement efficace de matière biologique provenant de colonies de différentes espèces bactériennes ; mais également dans leur capacité à réaliser un dépôt homogène sur plaque d'analyse MALDI-TOF. En effet, la performance d'identification des espèces bactériennes en spectrométrie de masse MALDI-TOF est directement liée à la qualité du dépôt de matière biologique sur la plaque d'analyse que ce soit en quantité de matière ou en homogénéité du dépôt.

Les différentes espèces ont donc fait l'objet d'une analyse en spectrométrie de masse MALDI-TOF afin de réaliser leur identification.

Le protocole d'analyse est celui décrit à l'exemple 1, incluant le dispositif de prélèvement.

Les matériaux testés sont présentés dans le **TABLEAU 2** ci-dessous :

**TABLEAU 2**

| | Diamètre mm | Longueur mm | Poids à sec mg | Poids (mg) eau +matériau | V1 air mm³ | V2 tot mm³ | porosité |
|---|---|---|---|---|---|---|---|
| Coton | 28 | 36 | 2412 | 24160 | 21748 | 22156 | 98% |
| PSU892 Copolymère PTE/PE | 2,5 | 5,9 | 6,1 | 27 | 21 | 29 | 74% |
| XA-20521-PS PE | 2,1 | 13,4 | 33,9 | 52 | 18 | 46 | 39% |

Dans le **TABLEAU 2,** sont indiquées les dimensions des échantillons de matériaux utilisés. Par ailleurs, les valeurs permettant de calculer la porosité, conformément à la méthode indiquée *supra*, ont également été indiquées.

Le matériau PSU892 est un matériau obtenu à partir de fibres bicomposants, constituées d'une enveloppe en polytéréphtalate d'éthylène et d'un coeur en polyester. Un tel matériau est commercialisé par la société POREX®. Il est par ailleurs décrit dans la demande de brevet WO-A-03/080904.

Le matériau XA-20521-PS est un matériau à partir de polyéthylène. Un tel matériau est commercialisé par la société POREX®, sous la dénomination commerciale NIBS.

Afin de réaliser les essais, trois espèces bactériennes différentes ont été utilisées :
- *Staphylococcus epidermidis* (souche ATCC 12228)
- *Klebsiella pneumoniae* (souche ATCC 13883)
- *Escherichia coli* (souche ATCC 25922)

Ces espèces ont été ensemencées chacune, sur une milieu de culture gélosé Columbia contenant 5% de sang de mouton (COS), commercialisée par la demanderesse sous la référence 43041.

Pour chaque espèce, une colonie est prélevée avec le dispositif de prélèvement selon l'invention et quatre dépôts sont réalisés sur quatre positions contiguës de la plaque d'analyse MALDI-TOF.

Les résultats sont présentés dans le **TABLEAU 3** ci-dessous :

**TABLEAU 3**

| Matériau | Espèce | Résultat d'analyse | Pourcentage d'identification |
|---|---|---|---|
| PSU 892 | *Staphylococcus Aureus* | 100 | 100% |
| | | 100 | |
| | | 100 | |
| | | 100 | |
| | *Klebsiella* | 99.99 | |
| | *pneumoniae* | 99.99 | |
| | | 99.99 | |
| | | 99.99 | |
| | *Escherichia* coli | 99.99 | |
| | | 99.99 | |
| | | 89.43 | |
| | | 95.14 | |
| | *Staphylococcus Aureus* | 99.99 | |
| | | 99.99 | |
| | | 99.99 | |
| | | 99.99 | |
| | *Klebsiella pneumoniae* | 99.99 | |
| Coton | | 99.99 | 100% |
| | | 99.99 | |
| | | 99.99 | |
| | *Escherichia* coli | 99.48 | |
| | | 98.41 | |
| | | 99.76 | |
| | | 99.91 | |
| | *Staphylococcus Aureus* | 99.99 | |
| | | 99.99 | |
| | | 99.99 | |
| | | 99.99 | |
| XA-20521-PS | *Klebsiella pneumoniae* | 99.99 | |
| | | 99.99 | 83,33% |
| | | 99.99 | |
| | | 99.99 | |
| | *Escherichia* coli | - | |
| | | 97.53 | |
| | | - | |
| | | 80.23 | |

On constate que pour les matériaux présentant une valeur élevée de porosité (supérieure à 70%), les performances d'identification sont excellentes, dans la mesure où l'ensemble des espèces sont correctement identifiées. C'est le cas avec le coton ou le matériau PSU 892.

Par contre, lorsqu'on utilise des matériaux présentant de plus faibles valeurs de porosité, les performances d'identification se trouvent impactées. Ainsi, avec le matériau XA-20521-PS qui a porosité de 39%, on constate que deux des quatre dépôts d'*Escherichia coli* n'ont pas permis l'identification de cette bactérie. Il ressort ainsi un taux d'identification de seulement 83,3%.

## Revendications

1. Embout apte à être emmanché sur le corps d'un dispositif de prélèvement, manuel ou automatisé, de matière biologique d'origine microbienne, comprenant :
a) une extrémité libre distale comprenant un moyen de prélèvement de matière biologique d'origine microbienne,
b) une extrémité libre proximale destinée à entrer en contact avec le corps dudit dispositif de prélèvement et permettre la fixation dudit embout sur ledit corps,
ledit embout étant **caractérisé en ce que** toute ou partie de l'extrémité libre distale est constituée d'un matériau fibreux présentant une porosité au moins égale à 30 %.

2. Embout selon la revendication 1, étant constitué entièrement d'un matériau fibreux présentant une porosité au moins égale à 30 %.

3. Embout selon la revendication 1 ou 2, dans lequel le matériau fibreux présente une porosité supérieure à 50%, préférentiellement supérieure à 70%

4. Embout selon l'une des revendications précédentes, dans lequel le matériau fibreux est pris dans le groupe comprenant : polyesters, polyéthylène, polytéréphtalate d'éthylène (PET), copolymère de PET et polyétylène, copolymère de PET/PET polyamide, coton.

5. Embout (10) selon l'une des revendications précédentes, présentant une forme globale sensiblement conique ou tronconique.

6. Embout (10) selon l'une des revendications précédentes, dans lequel le moyen de prélèvement est de forme globale cylindrique, tronconique ou sphérique.

7. Dispositif de prélèvement de matière biologique d'origine microbienne comprenant :
• un embout selon l'une des revendications 1 à 6
• un corps comportant au moins :
o une partie proximale servant au moins partiellement de zone de préhension dudit dispositif et
o une partie distale présentant une extrémité libre au bout de laquelle est fixé ledit embout.

8. Dispositif selon la revendication précédente, comprenant en outre un système d'éjection de l'embout.

9. Dispositif selon la revendication précédente, dans lequel le système d'éjection comporte une tige positionnée à l'intérieur dudit corps et mobile en translation.

10. Dispositif selon la revendication précédente, dans lequel ladite tige est mobile en translation au moyen d'un bouton poussoir.

11. Procédé de prélèvement de matière biologique comprenant les étapes suivantes :
a) Positionner un embout selon la revendication 1 à 6 sur la partie distale du dispositif de prélèvement selon l'une des revendications 7 ou 8,
b) Positionner le dispositif de prélèvement à proximité d'une colonie de matière biologique d'origine microbienne présente sur un milieu de culture afin de mettre en contact le moyen de prélèvement dudit embout avec la matière biologique,
c) Prélever tout ou partie de la matière biologique à l'aide du dispositif de prélèvement, de sorte que la matière biologique prélevée soit solidaire du moyen de prélèvement.

12. Procédé de préparation d'une plaque d'analyse pour une analyse microbiologique en spectrométrie de masse de type MALDI-TOF à partir d'une échantillon de matière biologique comprenant les étapes suivantes :
a) Positionner un embout selon la revendication 1 à 5 sur la partie distale du dispositif de prélèvement selon la revendication 6 ou 7,
b) Positionner le dispositif de prélèvement à proximité d'une colonie de matière biologique d'origine microbienne présente sur un milieu de culture afin de mettre en contact le moyen de prélèvement de l'embout avec la matière biologique,
c) Prélever tout ou partie de la matière biologique à l'aide du dispositif de prélèvement, de sorte que la matière biologique prélevée soit solidaire du moyen de prélèvement,
d) Déposer de manière uniforme la matière biologique prélevée sur au moins une zone d'analyse d'une plaque d'analyse de spectrométrie de masse de type MALDI-TOF, par mise en contact du moyen de prélèvement avec la surface de ladite au moins une zone d'analyse.

13. Procédé de préparation selon la revendication 11 ou 12, comportant en outre une étape supplémentaire e) d'éjection de l'embout de prélèvement.
